# EUROPEAN PATENT APPLICATION

(11) **EP 4 082 448 A1**
(43) Date of publication of application: **02.11.2022**
(21) Application number: 21170494.5
(22) Date of filing: 26.04.2021
(51) Int. Cl.: A61B 17/11

(54) **NERVE CONDUIT**

(71) Applicant: Tissium SA, 75012 Paris (FR)
(72) Inventor: Pereira, Maria, Paris (FR); Collin, Estelle, Paris (FR); Menand, Simon, Paris (FR)
(74) Representative: Graf von Stosch Patentanwaltsgesellschaft mbH

(57) **Abstract**

The invention relates to the field of nerve conduits as well as a use and a method of treating a nerve lesion using such nerve conduits. In particular, the invention relates to nerve conduits supporting the repair of nerve lesions by facilitating proper insertion and/or fixation of nerve ends into the nerve conduit. Accordingly, a nerve conduit (10) is suggested, comprising an elongate body (12) comprising a central portion (14) defining an inner cavity (16) and end portions (18) defining a respective opening (20) to the inner cavity (16) and arranged adjacent to the central portion (14) and at longitudinally opposing ends of the elongate body (12). According to the invention, a cross-sectional area of at least one opening (20) is larger than the cross-sectional area of the inner cavity (16) of the central portion (14).

## Description

### Technical field

The invention relates to nerve conduits as well as a use and a method of treating a nerve lesion using such nerve conduits. In particular, the invention relates to nerve conduits supporting the repair of nerve lesions by facilitating proper insertion and/or fixation of nerve ends into the nerve conduit.

### Technological Background

Upon injury of a person, tissue damage may involve one or more nerve lesions of the peripheral nerve system resulting in a partial sensory loss and/or impaired motor skills. Such injuries involving nerve damage particularly occur in the upper extremities, such as a hand or finger of a person, such that a person may experience a loss e.g. in tactile or haptic feedback and/or may have difficulties in controlling fine motor skills in the injured region, if the nerve lesion is not treated properly.

Current treatments of nerve lesions include coaptation of the nerve ends by various suturing techniques so as to provide a connection between the respective nerve ends in an essentially tensionless manner. In case a more severe defect is present, wherein the nerve ends are not directly adjacent to each other, a reconstruction may be required to overcome a corresponding gap. A reconstruction may be provided e.g. by an autologous or allogenic nerve graft. Alternatively, a reconstruction may be performed by providing a tubular structure so as to provide a nerve guide. Such tubular structure may be provided e.g. by autologous or allogenic venous structures or by artificially manufactured nerve conduits made of a biocompatible material. The use of a tubular structure may furthermore facilitate the repair of the lesion irrespective of the presence of a gap, e.g., by providing further mechanical support and structural stability, providing a tensionless repair, providing guidance to axonal growth limiting the risk of neuroma, reducing an inflammatory response to the site of the lesion, and/or limiting the extent of fibrous tissue development.

The tubular structures and, in particular, nerve conduits are generally formed as an essentially cylindrical shape extending in a longitudinal direction and defining an inner lumen or through hole from one end to the opposing end. The shape may also be formed as an irregular cylindrical shape, e.g. having a cross-section resembling a star-shape or snowflake-shape, be formed as an asymmetrical cylindrical shape, or may be formed as a shape having an essentially (rounded) rectangular cross-section. The dimensioning and shape are chosen so as to be compatible with the affected microstructure of the injured nerve tissue and surrounding tissue. Accordingly, in order to treat a nerve lesion, a nerve end is inserted into the nerve conduit via one end of the nerve conduit and the corresponding other nerve end is inserted into the nerve conduit via the opposing end of the nerve conduit. Both ends may then be fixed or coupled to the nerve conduit by means of suturing techniques or by applying or depositing a medical adhesive, for example. In the implanted state, the nerve conduit hence forms a nerve guide, wherein the continuous cylindrical shape of the nerve conduit and the inner lumen provides a directional path during neurogenesis.

### Summary of the invention

Starting from the known prior art there is a need to further facilitate the repair of nerve lesions.

According to the invention it has been recognized that the dimensional limitations and the continuous cylindrical shape of current nerve conduits render it difficult to insert nerve ends into the respective ends of the nerve conduit. The nerve ends are either pushed into the nerve conduit, risking potential nerve damage, or are pulled into the nerve conduit using microsutures, which may be cumbersome and may furthermore also be detrimental for the respective nerve ends. Furthermore, it has been found difficult to ensure that a predefined amount of a medical adhesive for the fixation of the nerve ends to the nerve guide is properly applied or deposited so as to enclose the respective nerve ends into the guide in an adequate manner. That is, a medical adhesive may not be homogeneously applied around the respective nerve end and/or an excess amount of medical adhesive may leak into a space between the respective nerve ends, potentially impairing nerve growth, or out of the conduit towards the surrounding tissue, potentially resulting in a loss of flexibility of the nerve conduit and/or negatively affecting said surrounding tissue.

It is hence an object of the present invention to further facilitate the repair of nerve lesions and, in particular, to ensure proper insertion and fixation of the respective nerve ends without the need of microsutures and/or minimizing the damage to the underlying nerve.

Said object is achieved by the independent claims. Preferred embodiments are depicted in the dependent claims, the description, and the Figures.

Accordingly, in a first aspect, a nerve conduit is suggested, which comprises an elongate body. The elongate body comprises a central portion defining an inner cavity and end portions defining a respective opening to the inner cavity and arranged adjacent to the central portion and at longitudinally opposing ends of the elongate body. According to the invention, a cross-sectional area of at least one opening is larger than the cross-sectional area of the inner cavity of the central portion.

The provision of the larger cross-sectional area at least at one end of the nerve conduit significantly facilitates the insertion of a respective nerve end of a nerve to be repaired. In particular, the larger cross-sectional area may provide a tolerance of an (unintended) offset of the insertion height of the nerve end with regard to the cross-sectional area of the inner cavity of the central portion during a repair procedure. Thereby, even if such offset is provided, it is ensured that the nerve end is received within the nerve conduit and (minor) adjustments may be performed without requiring a re-insertion of the nerve end. In addition, the larger cross-sectional area may serve as an optical guiding surface for a surgeon, thus further facilitating proper insertion of the respective nerve end. Thereby, proper insertion and placement of the nerve end into the nerve conduit, e.g. into the inner cavity, which may be particularly dimensioned to accommodate the nerve ends to be connected, may be achieved more easily and in a time-effective manner and without adversely affecting the respective nerve end.

At the same time the nerve conduit may still be configured according to the desirable or predefined dimensions and may provide a continuous guiding structure for neurogenesis. Modifications of the central portion are hence not necessary, such that a particular structural stability and dimensioning of the nerve conduit may be achieved. The lumen or interior of the elongate body, i.e. an inner wall of the central portion and/or end portion(s), may, however, comprise grooves, internal structures and/or holes. This may avoid a pressure build-up or a potential air bubble formation inside the nerve conduit and/or may improve the mechanical properties of the nerve conduit and/or promote nerve growth.

By the same token, depending on the material used for the nerve conduit, a predefined flexibility and/or resilience may be provided for at least the central portion, such that the end portions do not (significantly) affect the overall structural characteristics of the elongate body or the nerve conduit as a whole.

The larger cross-sectional area furthermore can assist in applying a medical adhesive towards or outside of the inner cavity, which may be particularly dimensioned to accommodate the nerve ends to be connected, via the corresponding opening, preferably only into a respective opening. For example, the larger cross-sectional area may prevent that the medical adhesive is inadvertently applied to an exterior surface of the nerve conduit and/or within the inner cavity of the central portion. In addition, this may provide that the medical adhesive is directed towards an interface between the opening and the inner cavity and/or that, when applying or inserting a predefined amount of medical adhesive, no significant amount of the medical adhesive leaks out of the nerve conduit and/or into the inner cavity of the central portion. In particular, the larger cross-sectional area facilitates applying a medical adhesive at the junction between the nerve and the nerve conduit, i.e. at the junction between the inserted nerve end and an edge of the opening at an end portion of the opening facing away from the central portion. The larger cross-sectional area may also be provided to receive a particular amount of the medical adhesive e.g. during inadequate application, such that it may indicate that the predefined amount of medical adhesive has been applied or it becomes immediately apparent that an application error occurs during the application of the medical adhesive. In other words, the larger cross-sectional area may also be configured to indicate that an excess amount of medical adhesive is being applied or the medical adhesive is not properly applied, such that the application needs to be interrupted or stopped.

The nerve conduit may have a variety of dimensions and shapes and may be configured to repair one or more nerve lesions. For example, directly adjacent nerves may be repaired by a single nerve conduit, wherein the inner cavity and the openings may e.g. provide a separating wall, such that two adjacent nerves may be respectively received in a separated compartment.

Accordingly, the elongate body may comprise multiple end portions and/or the openings at the longitudinal ends of the elongate body may comprise multiple openings at least one of its end portions, e.g. two distinct openings, or may be configured as a single opening defining two separate compartments (or receiving portions) for enabling insertion of and accommodating a respective nerve end. Such embodiment may have the advantage that only a single nerve conduit is required for multiple nerve lesions and/or nerve extensions and the procedure for repairing the nerve lesions or connecting the respective nerve ends may be facilitated. The elongate body may e.g. have a Y-shape.

Preferably, the central portion is formed as an essentially tubular shape, wherein the elongate body comprises two end portions. By such configuration the structural complexity of the overall nerve conduit may be reduced and the nerve conduit may be dimensioned so as to be adapted to the site of implantation and/or the requirements with respect to the particular nerve lesion. Such configuration may furthermore facilitate insertion of the respective nerve end at the opening having the larger cross-sectional area, since the elongate body may be configured only for a single nerve and inadvertent misplacement into a compartment not corresponding with the respective nerve end to be connected may be effectively avoided.

The tubular shape may provide that continuous outer dimensions may be provided, which may be advantageous for implanting the nerve conduit with regard to the surrounding tissue. Furthermore, the tubular or cylindrical shape may provide sufficient structural stability and may prevent sharp bends or kinking during tissue movement, i.e. compression or extension. The tubular shape may accordingly also provide that a homogeneous structure is provided, which reacts in a predefined manner along the entire central portion when forces act upon the central portion, e.g. upon impact.

The tubular structure may furthermore essentially correspond to the shape of the inner cavity, such that a continuous guiding structure for neurogenesis or nerve growth is provided, facilitating proper growth and connection of the respective nerve ends without providing an undesirable biasing and while achieving that the dimensioning of the inner cavity may be kept to a desirable and/or required minimum.

The openings and inner cavity may define a single lumen or continuous through hole. In other words, within the elongate body, a channel may be provided, which extends from one end of the elongate body to an opposing end of the elongate body in the longitudinal direction so as to provide a (fluid) connection or communication between the exterior of the respective ends via the inner cavity. The inner cavity should hence be understood as a central lumen, which extends and opens towards the respective end portions, such that a nerve end may be introduced into the lumen or through hole via the openings of the respective end portions. As described in the above, sections of the lumen or the elongate body may comprise holes, pores, grooves or particular geometries or surface irregularities, and/or a filler, which may facilitate the insertion, retention and/or growth of the respective nerve ends. One or more of these features may either be arranged in the interior or at the exterior of the elongate body or may extend between the interior and exterior of the elongate body. Furthermore, the elongate body and, in particular, the lumen may contain a drug, e.g. by means of coating or integrated in the material of the elongate body, which may be released over time and may e.g. facilitate nerve growth.

Preferably, the cross-sectional area of each of the openings is larger than the cross-sectional area of the inner cavity. This has the advantage that insertion and/or fixation of each of the respective nerve ends into the nerve conduit may be facilitated, thereby further supporting the repair procedure of the respective nerve lesion. The central portion may also be defined by an interface region between respective end portions and/or openings, wherein the longitudinal or axial extension of the central portion is essentially negligible compared with the corresponding extension of the end portions and/or openings, e.g. approximating zero or being less than e.g. 10 percent of the smallest axial extension of a respective opening or end portion. At least one of the openings may have a cross-sectional area being distinct from at least one other opening at an opposing end of the central portion and/or may have a cross-sectional area corresponding to the cross-sectional area of the inner cavity of the central portion, thereby forming an asymmetric nerve conduit.

Although the larger cross-sectional area of the at least one opening is already advantageous in terms of the insertion and/or fixation of the respective nerve end(s), the cross-sectional area of the at least one opening preferably increases in the longitudinal direction and away from the central portion. In other words, the opening may increase in a direction extending away from the central portion and along the longitudinal direction. In case of a tubular structure of the central portion, i.e. having a circular cross-section, a radial extension may hence be increased, preferably in all radial directions, whereas for an ellipsoid structure of the central portion, a radial extension may be increased in at least one direction, i.e. maintaining an overall ellipsoid shape of the opening or transforming to a more circular shape of the opening. Preferably, an increase of the opening of the respective end portion is provided directly starting or extending directly from the central portion.

The increasing opening may have a variety of shapes. Although a radial flare at the interface between the central portion and the respective end portion may be advantageous e.g. for handling of the nerve conduit during implantation, a gradually increasing and/or stepless increase of the opening may be preferred to facilitate insertion of the respective nerve end and/or to provide a guiding surface during the insertion of the respective nerve end. In other words, a gradual increase of the opening or cross-sectional area may direct the nerve end that is inserted into or towards the inner cavity and may avoid that a bending or sharp folding occurs by properly deflecting or guiding the respective nerve end. Hence, such gradual increase in the cross-sectional area may avoid a stump trauma or any other adverse effects of the respective nerve end potentially caused when the nerve end is brought into contact with an orthogonal and/or straight surface.

Accordingly, the at least one end portion and the corresponding opening are preferably formed as a rotationally symmetric shape along a longitudinal axis defined by the elongate body, wherein said shape is an essentially U-shape, sigmoidal shape, conical shape, concave shape, funnel shape, or parabolic shape.

As outlined above, such shapes may provide a gradual increase of the cross-sectional area. The preferred shapes are to be understood as a shape essentially extending from the central portion and as seen in a longitudinal sectional view of the elongate body. The particular shapes may facilitate that the respective nerve end is advanced into the opening and further into the inner cavity in an essentially stress-free and tensionless manner, since a contact between the nerve end and the inner surface of the end portion does not result in a significant bending of the nerve, but instead deflects the nerve end or tip thereof towards the advancing direction, i.e. towards the inner cavity. The hence provided guiding surface or structure facilitates the insertion procedure for a surgeon while at the same time the inner surface of the end portion does not adversely affect the respective nerve end during insertion.

Particularly advantageous embodiments in this regard include the funnel shape and/or the parabolic shape of the end portion and corresponding opening since these shapes may provide an essentially continuous and radially inward directing guiding surface, i.e. towards the inner cavity of the elongate body. Furthermore, these shapes provide further improved structural stability while avoid steps or sharp edges or large differences in the cross-sectional area towards the inner cavity or directly at the intersection between the inner cavity and the opening extending therefrom. The funnel shape may be formed e.g. essentially conical or have a parabolic extension so as to achieve e.g. the same maximum cross-sectional area compared with a conical shape yet in a corresponding smaller longitudinal extension and/or at a reduced angle with the central portion.

These above described preferred shapes and, in particular, the funnel and/or parabolic shape, furthermore facilitate the application of a medical adhesive, e.g., by maintaining the applied medical adhesive essentially outside of the inner cavity and/or by forming a junction or outer edge or rim to apply the medical adhesive, thereby ensuring that the respective nerve end is held within the nerve conduit and preventing a leakage towards the surrounding tissue and/or into the inner cavity of the central portion. The shapes may also define a limited reservoir or recess, preventing the medical adhesive from entering the nerve conduit and ensuring that the medical adhesive is held at said junction or edge region, e.g. in case of an application of excess medical adhesive or incorrect application.

The end portions may have been adapted to a particular dimension of a nerve lesion and the nerve conduit as a whole may be dimensioned to provide a bridging structure to a corresponding distance between respective nerve ends. Accordingly, the end portions of the elongate body may be configured differently so as to provide different characteristics, e.g. to accommodate different dimensions of the corresponding nerve ends. However, it is preferred that the end portions are equally formed. In such manner, the nerve conduit may be implanted essentially independent of the orientation of the respective end portions. In other words, by providing equally shaped end portions the nerve conduit may be configured to be essentially mirror symmetric and the nerve conduit may hence be placed in either direction, i.e. may also be placed in a 180 degrees inverted orientation.

The inner cavity and the openings are preferably formed by a single wall of the elongate body defining an inner diameter and outer diameter of the elongate body. In such a way, the structural complexity of the nerve conduit may be reduced while a more robust nerve conduit may be provided that responds to forces acting upon the elongate body in a predefined and/or expectable manner. For example, compressive and/or tensile forces acting on the elongate body may be better distributed (or absorbed) by the single wall, such that focal points of stress or tension may be avoided. Furthermore, depending on the material of the elongate body, a more homogeneous deflection of the nerve conduit may be provided, e.g., when the elongate body is formed of a wall having elastic and/or resilient characteristics.

For particular situations, the wall may have a (gradually) reduced thickness in at least one of the end portions and starting from the central portion towards the (longitudinal) outermost end of the respective end portion. In such a way, the larger cross-sectional area of the opening may be defined by the wall thickness.

However, the wall preferably comprises an essentially continuous thickness along the circumferential and longitudinal direction of the elongate body. This not only facilitates manufacturing of the nerve conduit, but also ensures that similar structural characteristics are provided along the longitudinal and circumferential direction. Adequate placement of the nerve conduit is hence also not dependent on an orientation of the nerve conduit in a circumferential direction.

In order to provide an essentially homogeneous inner cavity, which is advantageous to provide optimal nerve growth conditions and to provide an adequate guiding of the growing nerve end without undesirable biasing, the inner diameter and outer diameter of the central portion are preferably essentially continuous in the longitudinal direction of the elongate body.

The diameter of the at least one opening may increase in the longitudinal direction away from the central portion. In other words, the larger cross-sectional area of the corresponding opening may be defined by the inner diameter of the wall, wherein the diameter is enlarged towards the longitudinal end of the end portion facing away from the central portion and/or (directly) starting from the central portion. The increase in the opening is preferably gradual and homogeneous in all radial extensions. For example, the opening may have a circular cross-section along the entire longitudinal direction of the respective end portion, wherein the diameter of the circular shape is gradually increased. Such shape furthermore has the advantage that during implantation the placement of the nerve conduit is independent from the rotational orientation. Other shapes, such as ellipsoids, may, however, also be provided, wherein the (gradual) increase in the opening is provided by an increase in at least one radial extension.

The largest or maximum diameter of the at least one opening may be chosen such that the insertion of the respective nerve end is sufficiently supported while the overall thickness of the nerve conduit is maintained within physiologically acceptable limits. Hence, the maximum diameter is chosen to avoid that pressure and/or friction points are established with regard to the surrounding tissue and that the accommodated nerve end is sufficiently supported by the end portion.

Accordingly, a ratio between the maximum diameter of the at least one opening and the diameter of the inner cavity is preferably between 1,05:1,0 and 1,5:1,0, preferably between 1,05:1,0 and 1,2:1,0. It has been found that the difference in diameter and hence in cross-sectional area may thus be relatively small. The preferred ratio nevertheless achieves that a further improvement is provided with respect to the insertion and/or fixation of the respective nerve end. With a smaller ratio the application of a medical adhesive is furthermore facilitated, since this further reduces the risk of applying the adhesive between the respective nerve ends in the inner cavity of the central portion.

Preferably, the maximum (inner) diameter of the at least one opening is smaller than or corresponds to the outer diameter of the central portion. That is, seen from a longitudinal section, the opening does not exceed the outer diameter of the inner cavity in a radial direction. Thereby, depending e.g. on the thickness of the wall and the dimensioning of the inner cavity, the maximum diameter of the opening may hence be relatively small so as to maintain the overall appearance and/or to avoid large radial protrusions from the central portion.

The outer surface of the end portions extending from the central portion is preferably aligned with the outer surface of the central portion and/or is free of steps or edges with the outer surface of the central portion. This ensures that a continuous outer surface of the nerve conduit is provided and any edges or protrusions that may adversely affect the surrounding tissue, e.g. during movement of the nerve conduit, may be effectively avoided.

To accommodate for a variety of lengths to be bridged by the nerve conduit, the nerve conduit may be formed with different dimensions. The length of the central portion and the length of an end portion (or of each end portion) in the longitudinal direction may hence be different from each other and for each predefined configuration. Preferably, a ratio between the length of the central portion and the length of an end portion in the longitudinal direction is between 1,2:1,0 and 6,0:1,0, preferably between 1,2:1,0 and 1,4:1,0 or between 1,8:1,0 and 2,2:1,0 or between 4,4:1,0 and 5,4:1,0.

The ratio may be dependent on the overall length or absolute length of the nerve conduit or elongate body. Larger ratios may e.g. be provided for conduits having relatively small dimensions. In particular, the above larger ratios, i.e. of between 4,4:1,0 and 5,4:1,0 may be provided for nerve conduits having smaller dimensions, wherein hence relatively small end portions are provided compared with the central portion.

By the same token, a ratio between the length of the central portion and the length of the elongate body in the longitudinal direction may be between 0,3:1,0 and 0,8:1,0. Such ratio may increase with a decreasing overall length of the elongate body, such that the length of the elongate body may be primarily defined by the central portion and only includes small end portions for nerve conduits having relatively small or smallest dimensions.

The dependence of the length of the end portions, and the central portion, on the overall length may also provide that longer end portions may be provided when bridging longer distances between corresponding nerve ends, e.g. due to trauma, wherein the longer end portions may extend along a corresponding longer part of the respective nerve end. This may provide further structural stability, since these end portions may be configured to accommodate a (larger amount of) medical adhesive.

The length of the central portion in the longitudinal direction may be between 5 mm and 10 mm, preferably between 6,5 mm and 8,5 mm, wherein the length of the elongate body in the longitudinal direction may be between 7 mm and 25 mm, preferably between 9 mm and 22 mm, and/or wherein the length of (each of) the end portion(s) in the longitudinal direction may be between 1 mm and 8 mm, preferably between 1,3 mm and 6,5 mm. These dimensions have been found to be particularly advantageous to bridge nerve defects or lesions while facilitating nerve end insertion and/or fixation.

The diameter of the inner cavity may be between 1 mm and 12 mm, preferably between 1,5 mm and 6,5 mm, and/or the maximum diameter of the at least one opening may be between 1,5 mm and 7,5 mm, preferably between 1,75 mm and 6,5 mm. Again, such dimensions have been found to be particularly advantageous to provide the required support for the nerve ends and nerve growth while facilitating nerve end insertion and/or fixation.

The elongate body may be formed of a biocompatible material, an inert material, a bioimplantable material, and/or a biodegradable material. The material may be chosen so as to provide a predefined structural stability while essentially avoiding or at least reducing the inflammatory response of a patient to be treated. For example, a biocompatible material may be chosen, which is gradually degrading over time after implantation yet which may initially provide sufficient structural support to adequately repair a nerve lesion and ensure that the respective nerve ends are connected properly and with sufficient stability, e.g. during movement of the tissue.

The particular material may furthermore be chosen in order to facilitate or support nerve growth, for example, by comprising or otherwise incorporating or including a corresponding coating, e.g. with a biologically active agent and/or one or more neurotrophic factors. Other examples of such biologically active surface functionalities include, but are not limited to, e.g. anti-inflammatories, immunosuppressants, and neuroprotective agents. Biologically active agents may be surface bound and/or be entrapped in a structure defining the elongate body, e.g. the wall described in the above.

Preferably, the elongate body is formed of a polymer-based material, preferably an elastomer. This has the advantage that a plurality of manufacturing methods may be applied and/or particular characteristics often material may be provided based on e.g. a polymer unit. In particular, the polymer-based material may be a biocompatible material, which furthermore has elastic properties, such that, in the implanted states, the nerve conduit may adapt to tissue movement surrounding the repaired nerve lesion.

The elongate body may be formed of a polymerized and/or crosslinked polymer unit comprising an ester group component and an acid ester group component, the ester group component preferably being a polyol and the acid ester group component preferably being a polyacid.

The material being used for the central portion may be the same as the material of the end portions. Thereby, manufacturing may be further facilitated and structural characteristics of the elongate body may be essentially homogeneous along the longitudinal direction of the nerve conduit. In this matter, if the nerve conduit is configured accordingly, biodegradation (and/or bioresorption) may also occur in a predefined or expected manner. However, it may also be provided that at least one of the end portions is formed of a material being different from the central portion and/or the opposing end portion. Such configuration may be advantageous e.g. if one end portion has a particular functionality and may require different characteristics, e.g., if such an end portion is configured as a primary inlet for the application of a medical adhesive.

Preferably, the central portion and the end portions are integrally formed or formed of a single piece. In other words, the elongate body preferably does not require particular connections between the central portion and the end portions. Instead, it provides a material bonding, provided by the material off the central portion and/or the end portion, and corresponding structural integration of the respective elements. By providing the elongate body as a single piece, the robustness of the nerve conduit may be further improved since separate connections between components are effectively avoided.

The nerve conduit may be formed by a 3D-printing process. This is particularly advantageous when the material of the nerve conduit is polymer-based, wherein a curing of the material may be provided essentially instantaneously, for example using (UV) light. Furthermore, this provides an accuracy level that may not be (easily) achieved by means of extrusion and/or a dipping process. In particular, the 3-D printing process enables to obtain a particular shape of the nerve conduit, wherein, for example, the printing process may provide that particular biologically active agents are integrated in the 3-D structure according to a predefined pattern, e.g. within a mesh structure and/or in particular pockets or cavities formed by the 3-D structure. Thereby, orchestration and support of the nerve repair may be further improved and/or biodegradation may be achieved in a more controllable matter.

The above object is furthermore achieved by use of a nerve conduit described in the above for repairing, supporting, and/or guiding neural tissue, in particular for repairing a peripheral nerve lesion. Furthermore, the nerve conduit may be used in combination with a medical adhesive.

According to another aspect of the invention, a method for treating a peripheral nerve lesion is suggested, comprising the steps of:
providing a nerve conduit as described in the above;
inserting one end of the lesioned nerve into the nerve conduit via an opening having a larger cross-sectional area than the inner cavity of the central portion;
inserting another end of the lesioned nerve via an opening at an end potion at a longitudinally opposing end of the elongate body; and
securing the lesioned nerve ends within the elongate body.

Preferably, the securing of the lesioned nerve ends is performed by applying a medical adhesive outside of the inner cavity and/or within the openings via at least one of the openings.

### Brief description of the drawings

The present disclosure will be more readily appreciated by reference to the following detailed description when being considered in connection with the accompanying drawings in which:
Figure 1 shows a schematic depiction of a nerve conduit according to the invention in a longitudinal section;
Figure 2 shows a schematic depiction of a nerve conduit according to the invention in a longitudinal section according to Figure 1 with alternative end portions;
Figure 3 shows a schematic depiction of the nerve conduit according to Figure 2 in a side view from one end portion;
Figure 4 schematically shows the nerve conduit according to Figure 2 in a perspective view;
Figure 5 shows a schematic depiction of a nerve conduit according to the invention in a longitudinal section according to another embodiment; and
Figure 6 shows a schematic depiction of a nerve conduit according to the invention in a longitudinal section according to another embodiment.

### Detailed description of preferred embodiments

In the following, the invention will be explained in more detail with reference to the accompanying figures. In the Figures, like elements are denoted by identical reference numerals and repeated description thereof may be omitted in order to avoid redundancies.

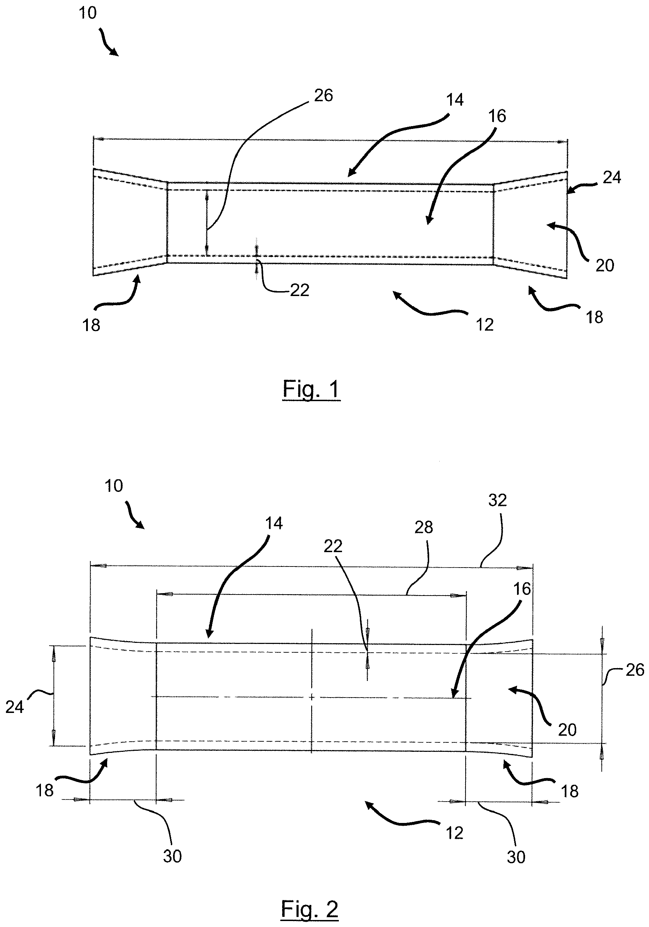

In Figure 1 a nerve conduit 10 according to the invention is schematically shown along a longitudinal section. The nerve conduit 10 comprises an elongate body 12 having a central portion 14, which defines an inner cavity 16. In the present embodiment the inner cavity 16 is essentially defined by a wall 22. The wall 22 has a continuous tubular or cylindrical shape in the longitudinal direction of the elongate body 12 and has a continuous thickness both in the circumferential and longitudinal direction. Thereby, the inner cavity 16 is also formed as a cavity having a cylindrical shape having essentially continuous dimensions. However, it will be understood that embodiments of the invention are not limited to such configuration and varying wall thicknesses and/or alternative shapes off the central portion 14 and inner cavity 16 may also be provided.

The nerve conduit 10 or elongate body 12 thereof, according to the present exemplary embodiment, comprises two end portions 18, which are arranged at longitudinally opposing ends of the elongate body 12 and are arranged directly adjacent to the central portion 14. As indicated in the schematic depiction according to Figure 1, the outer surfaces of both end portions 18 are aligned or flush with the outer surface of the central portion 14, such that a homogeneous and stepless outer surface is provided that is free of sharp edges, recesses, and/or protrusions potentially adversely affecting the surrounding tissue in the implanted state of the nerve conduit 10.

Both end portions 18 define an opening 20 to the inner cavity 16, such that a continuous through hole is provided from one end of the elongate body 12 to the opposing end of the elongate body 12 and a fluid communication between the inner cavity 16 and the exterior of the elongate body 12 is provided. Accordingly, nerve ends of a nerve to be repaired, e.g. after trauma resulting in a nerve lesion, may be inserted into the inner cavity 16 via a respective opening 20 of the corresponding end portion 18. The end portions 18 according to the present embodiment are equally shaped and dimensioned, such that during implantation a reversed orientation of the nerve conduit 10 does not affect the procedure.

The opening 20 of each of the end portions 18 increases along the longitudinal direction of the elongate body 12 in a direction away from the central portion 14. In other words, each opening 20 or radial extension thereof (gradually) increases starting from or extending from the central portion 14. In the present exemplary embodiment, a gradual increase of the openings 20 is provided by a conical shape of the end portions 18 or wall 22 thereof. The continuous wall thickness and the shapes of the central portion 14 and the end portions 18 hence provide that the cross-sectional area of each of the openings 20 is larger than the cross-sectional area of the inner cavity 16 of the central portion 14. Thereby, insertion of a respective nerve end into the inner cavity 16 and the application of a medical adhesive outside of the inner cavity 16, in particular at a junction or edge region of the opening, may be facilitated.

Both the inner cavity 16 and the openings 20 comprise an essentially circular shape in a cross-sectional view, wherein the diameter 26 of the inner cavity 16 is essentially continuous. The diameter of each of the openings 20 at the interface with the central portion 14 or inner cavity 16 essentially corresponds to the diameter 26 of the inner cavity 16. However, the diameter of each of the openings 20 increases along a longitudinal direction towards the outermost end of the elongate body 12 and end portion 18 respectively facing away from the central portion 14. In the present embodiment, the maximum diameter 24 and/or radial extension of each of the openings 20, and thereby the largest cross-sectional area, is hence provided at the longitudinally opposing and outermost ends of the elongate body 12. Having the largest cross-sectional area at the outermost end of the elongate body 16 further facilitates the insertion of a respective nerve end and/or the application of a medical adhesive outside of the inner cavity 16 or at the interface between the inner cavity 16 and the respective opening 20 or at the interface between the respective opening 20 and the respective inserted nerve end.

In Figure 2, a nerve conduit 10 is shown, which essentially corresponds to the embodiment according to Figure 1. However, this embodiment differs with regard to the shape of the end portions 18. In this embodiment, the end portions 18 are funnel-shaped and define a radially outward curvature or parabolic extension starting from the end facing the central portion 14 and extending towards the outermost end of the elongate body 12. In this manner, a larger opening 20 may be provided at the outermost end without requiring an increase in the length of the end portion 18 in the longitudinal direction and without requiring large angular offsets at the interface between the central portion 14 and the respective end portion 18, thereby also improving the structural stability of the nerve conduit 10. Furthermore, such shape may be advantageous to avoid sharp bends of the respective nerve end upon insertion and by providing a gradual guiding surface towards the inner cavity 16.

As indicated in Figure 2 the maximum diameter 24 of the openings 20 is larger than the (constant) diameter 26 of the inner cavity 16. In addition, Figure 2 demonstrates possible ratios between these diameters 24, 26 as well as length ratios between the corresponding portions 14, 18 of the elongate body 12.

For example, the ratio between the maximum diameter 24 of the opening 20 and the diameter 26 of the inner cavity 16 may be between 1,05:1,0 and 1,2:1,0. In a preferred embodiment, the maximum diameter 24 of the openings 20 may be between 1,75 mm and 6,5 mm or up to 12 mm while the diameter 26 of the inner cavity 16 may be between 1,5 mm and 6 mm or up to 11 mm. Accordingly, the cross-sectional area of the maximum diameter 26 of the openings may be between about 2,4 mm² and about 33,2 mm² and the cross-sectional area of the diameter 26 of the inner cavity 16 may be between about 1,75 mm² and about 28,3 mm².

In a particular example, the maximum diameter 24 may e.g. comprise 1,75 mm or 3,5 mm while the diameter 26 may accordingly comprise e.g. 1,5 mm and 3 mm, respectively, such that corresponding cross-sectional areas of the maximum diameter 24 may be about 2,4 mm² and about 9,6 mm² and corresponding cross-sectional areas of the diameter 26 of the inner cavity 16 may be about 1,75 mm² and about 7,1 mm² and a ratio of about 1,17:1,0 is obtained.

Such ratios have been found to be particularly advantageous to facilitate the insertion of respective nerve ends and/or apply a medical adhesive into the inner cavity 16 while limiting the radial extension of the nerve conduit. It is to be understood that embodiments may comprise the above configuration, but are not limited to the above exemplary dimensions and other dimensions or ratios may be provided as long as they are within the indicated preferred range.

Alternatively, or in addition, a ratio between the length 28 of the central portion 14 and the length 30 of an end portion 18 in the longitudinal direction may preferably be between 1,2:1,0 and 1,4:1,0 or between 1,8:1,0 and 2,2:1,0 or between 4,4:1,0 and 5,4:1,0. By the same token, a ratio between the length 28 of the central portion 14 and the length 32 of the elongate body 12 in the longitudinal direction is preferably between 0,3:1,0 and 0,8:1,0. For example, the length 28 of the central portion 14 in the longitudinal direction may preferably be between 6,5 mm and 8,5 mm, wherein the length 32 of the elongate body in the longitudinal direction is preferably between 9 mm and 22 mm, and wherein the length 30 of the end portion 18 in the longitudinal direction is preferably between 1,3 mm and 6,5 mm.

In one exemplary embodiment, the length 28 of the central portion 14 may be e.g. 8 mm and the length 30 of the respective end portions 18 may be e.g. 6 mm, such that the total length 32 of the elongate body 12 may be e.g. 20 mm, resulting in a ratio of 1,33:1,0 and 0,4:1,0 between the length 28 of the central portion 14 and the length 30 of the respective end portion 18 and in a ratio of 0,4:1,0 between the length 28 of the central portion 14 and the length 32 of the elongate body 12. Again, it is to be understood that embodiments may comprise the above configuration, but are not limited to the above exemplary dimensions and other dimensions or ratios may be provided as long as they are within the indicated preferred range.

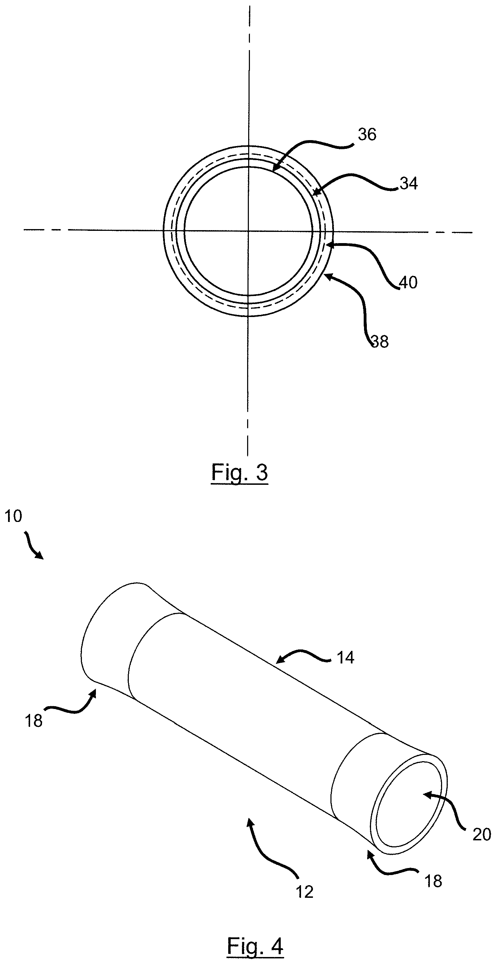

In Figure 3 the different cross-sectional areas are shown in a side view of the nerve conduit as seen from one outermost end of the elongate body depicted in Figure 2. Accordingly, the cross-sectional area 34 of the openings is larger than the cross-sectional area 36 of the inner cavity, which is indicated with the corresponding different diameters. In other words, the wall of the elongate body at the region defining the maximum diameter of the opening is radially offset to the inner wall of the central portion defining the inner cavity. As described above, the cross-sectional area of or at the maximum diameter of the openings may be e.g. between about 2,4 mm² and about 33,2 mm² and the cross-sectional area of the inner cavity 16 may be between about 1,75 mm² and about 28,3 mm². As indicated with the dashed line 40, the outer surface 38 of the end portion does not extend beyond the outer surface 40 of the central portion.

Although Figure 3 comprises the cross-sectional area as a circular or round shape, it will be understood that the embodiments depicted in the Figures are not limited to such shape and other shapes such as ellipsoids may be provided, wherein the difference in the cross-sectional area 34 of the opening(s) and the cross-sectional area 36 of the inner cavity may be provided by an extension offset in at least one radial direction.

Figure 4 schematically shows a nerve conduit 10 according to the embodiment shown in Figures 2 and 3 in a perspective view. From this Figure, it may be appreciated that the parabolic shape of the respective end portions 18 achieves that the opening 20 is increased towards the respective outermost ends of the elongate body 12 and provides a guiding surface facilitating insertion of the respective nerve ends into the inner cavity of the central portion 14. At the same time, the gradual increase of the opening 20 and the parabolic shape allow that the radial extension and the length of the end portions 18 may be kept to a minimum required and do not considerably affect the exterior and structural stability of the nerve conduit 10. Thereby, the repair of nerve lesions is facilitated by the advantageous configuration and dimensioning of the nerve conduit 10 according to the invention without adversely affecting the surrounding tissue in the implanted state of the nerve conduit 10.

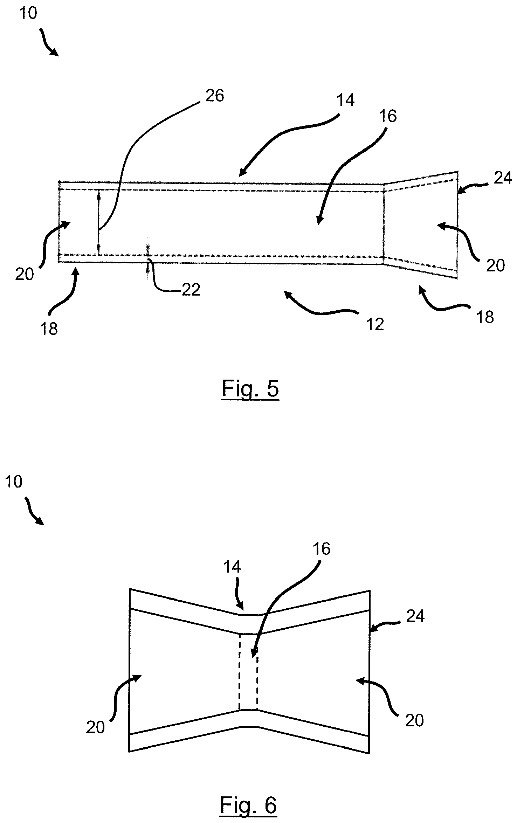

In Figure 5, a nerve conduit 10 is schematically depicted according to an embodiment having distinct end portions 18 and openings 20, wherein only one opening 20 has a larger cross-sectional area compared with the central portion 14, i.e. having a larger maximum diameter 24 than the diameter 26 of the inner cavity 16. The nerve conduit 10 is hence formed as an asymmetric nerve conduit 10, which may particularly facilitate inserting a thicker nerve end at the opening 20 having the larger cross-sectional area.

An embodiment of a nerve conduit 10 having opposing end portions 18 and openings 20 that are formed of essentially the same shape, e.g. a funnel shape, is shown in Figure 6, wherein the central portion 14 and inner cavity 16 are defined as an interface region between the respective openings 20, as indicated with the dashed lines. The interface region may e.g. be formed by an adjacent end of the respective openings 20 or end portions 18, which are adjoined and wherein the interface region may be rounded or formed so as to avoid significant steps between the respective end portions 18. In other words, the interface region may be formed to provide a continuous inner surface and outer surface of a wall essentially forming the nerve conduit 10.

According to an embodiment, the longitudinal or axial extension of the central portion 14 is essentially negligible compared with the corresponding extension of the end portions 18 or openings 20, e.g. approximating zero or being less than e.g. 10 percent of the smallest axial extension of a respective opening 20 or end portion 18.

It will be obvious for a person skilled in the art that these embodiments and items only depict examples of a plurality of possibilities. Hence, the embodiments shown here should not be understood to form a limitation of these features and configurations. Any possible combination and configuration of the described features can be chosen according to the scope of the invention.

The invention is furthermore depicted in the following items:
1. A nerve conduit (10), comprising an elongate body (12) comprising a central portion (14) defining an inner cavity (16) and
   end portions (18) defining a respective opening (20) to the inner cavity (16) and arranged adjacent to the central portion (14) and at longitudinally opposing ends of the elongate body (12),
   wherein a cross-sectional area of at least one opening (20) is larger than the cross-sectional area of the inner cavity (16) of the central portion (14).
2. The nerve conduit (10) according to item 1, wherein the central portion (14) is formed as an essentially tubular shape and the elongate body (12) comprises two end portions (18).
3. The nerve conduit according to item 1 or 2, wherein the openings (20) and inner cavity (16) define a single lumen or continuous through hole.
4. The nerve conduit (10) according to any of the preceding items, wherein the cross-sectional area of each of the openings (20) is larger than the cross-sectional area of the inner cavity (16) or wherein the cross-sectional area of an opening (20) of only one end portion (18) is larger than the cross-sectional area of the inner cavity (16).
5. The nerve conduit (10) according to any of the preceding items, wherein the cross-sectional area of the at least one opening (20) increases in the longitudinal direction and away from the central portion (14).
6. The nerve conduit (10) according to item 5, wherein the at least one end portion (18) and the corresponding opening (20) are formed as a rotationally symmetric shape along a longitudinal axis defined by the elongate body (12), said shape being an essentially U-shape, sigmoidal shape, conical shape, concave shape, funnel shape, or parabolic shape.
7. The nerve conduit (10) according to any of the preceding items, wherein the end portions (18) are equally formed.
8. The nerve conduit (10) according to any of the preceding items, wherein the inner cavity (16) and the openings (20) are formed by a single wall (22) of the elongate body (12) defining an inner diameter and outer diameter of the elongate body (12).
9. The nerve conduit (10) according to item 8, wherein the wall (22) comprises an essentially continuous thickness along the circumferential and longitudinal direction of the elongate body (12).
10. The nerve conduit (10) according to item 8 or 9, wherein the inner diameter and outer diameter of the central portion (14) are essentially continuous in the longitudinal direction of the elongate body (12).
11. The nerve conduit (10) according to any of items 8 to 10, wherein the diameter of the at least one opening (20) increases in the longitudinal direction away from the central portion (14).
12. The nerve conduit (10) according to item 11, wherein a ratio between the maximum diameter (24) of the at least one opening (20) and the diameter (26) of the inner cavity (16) is between 1,05:1,0 and 1,5:1,0, preferably between 1,05:1,0 and 1,2:1,0.
13. The nerve conduit (10) according to item 11 or 12, wherein the maximum diameter (24) of the at least one opening is smaller than or corresponds to the outer diameter of the central portion (14).
14. The nerve conduit (10) according to any of the preceding items, wherein the outer surface (38) of the end portions (18) extending from the central portion (14) is aligned with the outer surface (40) of the central portion (14) and/or is free of steps or edges with the outer surface (40) of the central portion (14).
15. The nerve conduit (10) according to any of the preceding items, wherein a ratio between the length (28) of the central portion (14) and the length (30) of an end portion (18) in the longitudinal direction is between 1,2:1,0 and 6,0:1,0, preferably between 1,2:1,0 and 1,4:1,0 or between 1,8:1,0 and 2,2:1,0 or between 4,4:1,0 and 5,4:1,0.
16. The nerve conduit (10) according to any of the preceding items, wherein a ratio between the length (28) of the central portion (14) and the length (32) of the elongate body (12) in the longitudinal direction is between 0,3:1,0 and 0,8:1,0.
17. The nerve conduit (10) according to any of the preceding items, wherein the length (28) of the central portion (14) in the longitudinal direction is between 5 mm and 10 mm, preferably between 6,5 mm and 8,5 mm, wherein the length (32) of the elongate body (12) in the longitudinal direction is between 7 mm and 25 mm, preferably between 9 mm and 22 mm, and/or wherein the length (30) of the end portion (18) in the longitudinal direction is between 1 mm and 8 mm, preferably between 1,3 mm and 6,5 mm.
18. The nerve conduit (10) according to any of the preceding items, wherein the diameter (26) of the inner cavity (16) is between 1 mm and 12 mm, preferably between 1,5 mm and 6,5 mm, and/or wherein the maximum diameter (24) of the at least one opening (20) is between 1,5 mm and 11 mm, preferably between 1,75 mm and 6,5 mm.
19. The nerve conduit (10) according to any of the preceding items, wherein the elongate body (12) is formed of a biocompatible material, an inert material, a bioimplantable material, and/or biodegradable material.
20. The nerve conduit (10) according to any of the preceding items, wherein the elongate body (12) is formed of a polymer-based material, preferably an elastomer.
21. The nerve conduit (10) according to any of the preceding items, wherein the central portion (14) and the end portions (18) are integrally formed or formed of a single piece.
22. The nerve conduit (10) according to any of the preceding items, wherein the elongate body (12) is formed of a polymerized and/or crosslinked polymer unit comprising an ester group component and an acid ester group component, the ester group component preferably being a polyol and the acid ester group component preferably being a polyacid.
23. The nerve conduit (10) according to any of the preceding items formed by a 3D-printing process.
24. Use of a nerve conduit (10) according to any of the preceding items for repairing, supporting, and/or guiding neural tissue, in particular for repairing a peripheral nerve lesion.
25. Use of a nerve conduit (10) according to item 24 in combination with a medical adhesive.
26. A method of treating a peripheral nerve lesion, comprising the steps of:
   - providing a nerve conduit (10) according to any of the preceding claims;
   - inserting one end of the lesioned nerve into the nerve conduit (10) via an opening (20) having a larger cross-sectional area than the inner cavity (16) of the central portion (14);
   - inserting another end of the lesioned nerve via an opening (20) at an end potion (18) at a longitudinally opposing end of the elongate body (12); and
   - securing the lesioned nerve ends within the elongate body (12).
27. The method according to item 26, wherein the securing of the lesioned nerve ends is performed by applying a medical adhesive outside of the inner cavity (16) and/or into the openings (20) via at least one of the openings (20) and/or around the openings (20).

### List of reference numerals

- 10: Nerve conduit
- 12: Elongate body
- 14: Central portion
- 16: Inner cavity
- 18: End portion
- 20: Opening
- 22: Wall
- 24: Maximum diameter of opening
- 26: Diameter of inner cavity
- 28: Length of central portion
- 30: Length of end portion
- 32: Length of elongate body
- 34: Cross-sectional area of opening
- 36: Cross-sectional area of inner cavity
- 38: Outer surface of end portion
- 40: Outer surface of central portion

## Claims

1. A nerve conduit (10), comprising an elongate body (12) comprising a central portion (14) defining an inner cavity (16) and
end portions (18) defining a respective opening (20) to the inner cavity (16) and arranged adjacent to the central portion (14) and at longitudinally opposing ends of the elongate body (12),
wherein a cross-sectional area of at least one opening (20) is larger than the cross-sectional area of the inner cavity (16) of the central portion (14).

2. The nerve conduit (10) according to claim 1, wherein the central portion (14) is formed as an essentially tubular shape and the elongate body (12) comprises two end portions (18) and/or wherein the openings (20) and inner cavity (16) define a single lumen or continuous through hole.

3. The nerve conduit (10) according to any of the preceding claims, wherein the cross-sectional area of each of the openings (20) is larger than the cross-sectional area of the inner cavity (16) or wherein the cross-sectional area of an opening (20) of only one end portion (18) is larger than the cross-sectional area of the inner cavity (16).

4. The nerve conduit (10) according to any of the preceding claims, wherein the cross-sectional area of the at least one opening (20) increases in the longitudinal direction and away from the central portion (14).

5. The nerve conduit (10) according to claim 4, wherein the at least one end portion (18) and the corresponding opening (20) are formed as a rotationally symmetric shape along a longitudinal axis defined by the elongate body (12), said shape being an essentially U-shape, sigmoidal shape, conical shape, concave shape, funnel shape, or parabolic shape.

6. The nerve conduit (10) according to any of the preceding claims, wherein the inner cavity (16) and the openings (20) are formed by a single wall (22) of the elongate body (12) defining an inner diameter and outer diameter of the elongate body (12), wherein the wall (22) preferably comprises an essentially continuous thickness along the circumferential and longitudinal direction of the elongate body (12).

7. The nerve conduit (10) according to claim 6, wherein the inner diameter and outer diameter of the central portion (14) are essentially continuous in the longitudinal direction of the elongate body (12).

8. The nerve conduit (10) according to any of claims 6 or 7, wherein the diameter of the at least one opening (20) increases in the longitudinal direction away from the central portion (14), wherein a ratio between the maximum diameter (24) of the at least one opening (20) and the diameter (26) of the inner cavity (16) is preferably between 1,05:1,0 and 1,5:1,0, more preferably between 1,05:1,0 and 1,2:1,0.

9. The nerve conduit (10) according to claim 8, wherein the maximum diameter (24) of the at least one opening is smaller than or corresponds to the outer diameter of the central portion (14).

10. The nerve conduit (10) according to any of the preceding claims, wherein the outer surface (38) of the end portions (18) extending from the central portion (14) is aligned with the outer surface (40) of the central portion (14) and/or is free of steps or edges with the outer surface (40) of the central portion (14).

11. The nerve conduit (10) according to any of the preceding claims, wherein a ratio between the length (28) of the central portion (14) and the length (30) of an end portion (18) in the longitudinal direction is between 1,2:1,0 and 6,0:1,0, preferably between 1,2:1,0 and 1,4:1,0 or between 1,8:1,0 and 2,2:1,0 or between 4,4:1,0 and 5,4:1,0.

12. The nerve conduit (10) according to any of the preceding claims, wherein a ratio between the length (28) of the central portion (14) and the length (32) of the elongate body (12) in the longitudinal direction is between 0,3:1,0 and 0,8:1,0.

13. The nerve conduit (10) according to any of the preceding claims, wherein the length (28) of the central portion (14) in the longitudinal direction is between 5 mm and 10 mm, preferably between 6,5 mm and 8,5 mm, wherein the length (32) of the elongate body (12) in the longitudinal direction is between 7 mm and 25 mm, preferably between 9 mm and 22 mm, and/or wherein the length (30) of the end portion (18) in the longitudinal direction is between 1 mm and 8 mm, preferably between 1,3 mm and 6,5 mm.

14. The nerve conduit (10) according to any of claims 1 to 10, wherein the central portion (14) is defined by an interface region between respective end portions (18) and/or corresponding openings (20), wherein the extension of the central portion (14) in the longitudinal direction is preferably less than about 10 percent of the smallest extension of a respective opening (20) and/or end portion (18) in the longitudinal direction.

15. The nerve conduit (10) according to any of the preceding claims, wherein the diameter (26) of the inner cavity (16) is between 1 mm and 12 mm, preferably between 1,5 mm and 6,5 mm, and/or wherein the maximum diameter (24) of the at least one opening (20) is between 1,5 mm and 11 mm, preferably between 1,75 mm and 6,5 mm.
